(19) Europäisches Patentamt
European Patent Office
Office européen des brevets

(11) Veröffentlichungsnummer: **0 305 843**
**A2**

# (12) EUROPÄISCHE PATENTANMELDUNG

(21) Anmeldenummer: **88113610.5**

(22) Anmeldetag: **22.08.88**

(51) Int. Cl.4: **A61K 31/70 , C07H 19/067 , C07H 19/167**

(30) Priorität: **01.09.87 DE 3729066**
**02.03.88 DE 3806633**

(43) Veröffentlichungstag der Anmeldung:
**08.03.89 Patentblatt 89/10**

(84) Benannte Vertragsstaaten:
**AT BE CH DE ES FR GB GR IT LI NL SE**

(71) Anmelder: **BAYER AG**
**Konzernverwaltung RP Patentabteilung**
**D-5090 Leverkusen 1 Bayerwerk(DE)**

(72) Erfinder: **Barth, Wolfgang, Dr.**
**Ludwig-Erhard-Weg 26**
**D-5600 Wuppertal 1(DE)**
Erfinder: **Häbich, Dieter, Dr.**
**Krummacherstrasse 82**
**D-5600 Wuppertal 1(DE)**
Erfinder: **Paessens, Arnold, Dr.**
**Stresemannstrasse 56**
**D-5657 Haan 1(DE)**
Erfinder: **Streissle, Gert, Dr.**
**Gellertweg 22**
**D-5600 Wuppertal 1(DE)**

## (54) Antivirales Mittel.

(57) Die Erfindung betrifft 1-(2,3-Anhydro-β-lyxofuranosyl)-purin- oder 9-pyrimidinnucleoside der Formel I

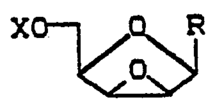

(I)

in der X und R die in der Beschreibung angegebene Bedeutung haben, zur Anwendung in einem Verfahren zur therapeutischen Behandlung des menschlichen oder tierischen Körpers, antivirale Arzneimittel, enthaltend die Verbindungen der Formel I und die Verwendung dieser Verbindungen zur Herstellung von antiviralen Arzneimitteln.

EP 0 305 843 A2

## Antivirales Mittel

Die Erfindung betrifft die Verwendung von 1-(2,3-Anhydro-β-lyxofuranosyl)purin- oder 9-pyrimidinnucleosiden als antivirale Mittel in der Human- und Tiermedizin.

Es ist bekannt, daß $2',3'$-Didesoxynucleoside gegen Retroviren wirksam sind. [H. Mitsuya, S. Broder, Proc. nat. Acad. Sci. USA 38, 1911-1915 (1986); EP-A 206 497].

Es wurde nun gefunden, daß Epoxide der allgemeinen Formel (I)

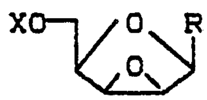

(I)

in welcher
R für eine Gruppe der Formel

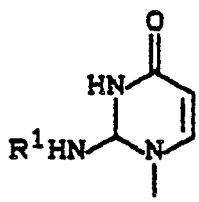

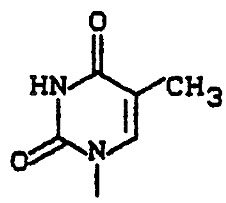

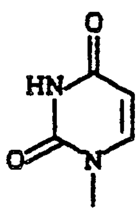

,

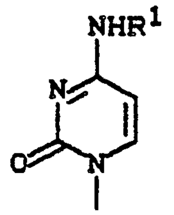

oder

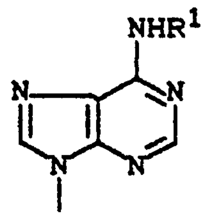

steht,

wobei
$R^1$ Wasserstoff oder Acetyl bedeutet,
und
X für Wasserstoff oder
für geradkettiges $C_1$-$C_7$-Alkylcarbonyl, Isopropylcarbonyl, tert. Butylmethylcarbonyl oder Benzoyl steht,
oder ein pharmazeutisch verträgliches Derivat hiervon,
starke antivirale Wirkung gegen Retroviren aufweisen.

Die erfindungsgemäß zu verwendenden Epoxide sind durch die Formel (I) allgemein definiert.

In dieser Formel steht vorzugsweise
R für eine Gruppe der Formel

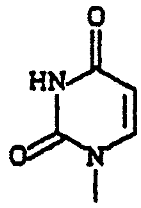 , 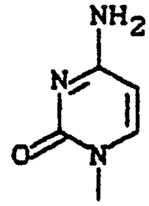 , 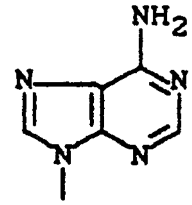 ,

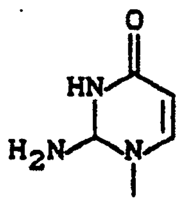 oder 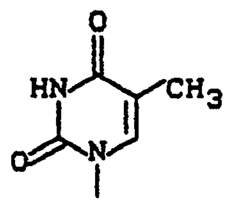 steht,

und
X für Wasserstoff oder Acetyl, Propionyl, Propylcarbonyl, Isopropylcarbonyl oder Butylcarbonyl.

Besonders bevorzugt sind Verbindungen der allgemeinen Formel (I),
in welcher
R für eine Gruppe der Formel

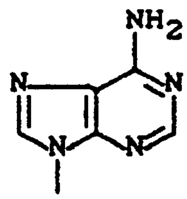 steht,

und
X für wasserstoff steht.

Ganz besonders bevorzugt ist 9-(2,3-Anhydro-β-D-lyxofuranosyl)adenin.

Ebenso verwendbar sind auch pharmazeutisch verträgliche Derivate der Verbindungen der allgemeinen Formel (I). Hierzu gehören im allgemeinen pharmazeutisch verträgliche Salze, Ester, ein Salz eines solchen Esters, oder aber Verbindungen, die bei Applikation die erfindungsgemäß zu verwendenden Verbindungen als Stoffwechselprodukte oder Abbauprodukte bereitstellen, auch "Prodrugs" genannt. Ester können hierbei Veresterungsprodukte der -OH-Gruppe (X = H) der erfindungsgemäß zu verwendenden Verbindungen der allgemeinen Formel (I) mit Carbonsäuren, Sulfonsäuren oder Phosphorsäuren sein. Insbesondere zu nennen seien Carbonsäureester abgeleitet von $C_1$-$C_{10}$-Alkylcarbonsäuren, gegebenenfalls substituierten Benzoesäuren, $C_1$-$C_6$-Alkylsulfonsäuren oder auch Mono-, Di- oder Triphosphatester.

Als Salze können Salze mit üblichen Basen genannt werden, wie beispielsweise Alkalimetallsalze (z.B. Natrium-oder Kaliumsalze), Erdalkalisalze (z.B. Calcium- oder Magnesiumsalze) oder Ammoniumsalze abgeleitet von Ammoniak oder organischen Aminen (wie beispielsweise Diethylamin, Triethylamin, Ethyldiisopropylamin, Dibenzylamin, Prokain, Dibenzylamin, N-Methylmorpholin, Dehydroabietylamine, 1-Ephenamin oder Methyl-piperidin).

Die erfindungsgemäß zu verwendenden Wirkstoffe sind bekannt:

a) (Uracilderivat): J.F. Codington, R. Fecher, J.J. Fox, J. Org. Chem. 27 (1962), 163, M. Hirata, Chem. Pharm, Bull. 16 (1968), 430.

b) Cytosinderivat): D.H. Hollenberg, K.A. Watanabe, J.J. Fox, J. Med. Chem. 20 (1977), 113.

c) (Adeninderivat): R. Mengel, M. Bartke, Angew, Chem. 90 (1978), 725 und M.J. Robins, Y. Fouron, R. Mengel, J. Org. Chem. 39 (1974), 1564.

d) (Isocytosinderivat): M. Hirata, Chem. Pharm. Bull. 16 (1968), 430

e) (Thyminderivat): GB 1049312 und A.V. Papchikhin et. al., Bioorg. Khim, 11 (1985), 1367.

Es wurde im Rahmen von Untersuchungen, die zu der vorliegenden Erfindung führten, überraschend gefunden, daß die erfindungsgemäß zu verwendenden Verbindungen der allgemeinen Formel (I) eine außerordentlich starke Wirkung gegen Retroviren besitzen. Dies wird durch die weiter unten angegebenen

Versuchsdaten am Beispiel des Visna-Virus für 9-(2,3-Anhydro-β-D-lyxofuranosyl)adenin in Zellkulturen belegt. Das Visna-Virus und das HIV-Virus (Virus der humanen Immundefiziens) gehören beide zu den Lentiviren, die zu der Gruppe der Retroviren gehören.

In Zellkulturen, die mit Visna-Virus infiziert sind, treten 5 bis 10 Tage nach der Infektion ausgeprägte virusinduzierte, zytopathische Effekte auf. Durch Behandlung der infizierten Zellkulturen mit 9-(2,3-Anhydro-β-D-lyxofuranosyl)adenin konnte das Auftreten dieser zytopathischen Effekte verhindert werden. Der Visna-Virus-Test wurde nach der Methode von O. Narayan et al., Journal of Infectious Dieseases 135, 5, 1977, 800-806 durchgeführt. Dazu wurde 9-(2,3-Anhydro-β-D-lyxofuranosyl)adenin - in Produktionsmedium gelöst - in nicht zytotoxischen Konzentrationen in 96er Microtiterplatten verdünnt. Anschließend wurden Fibroblastenzellen vom Schaf ($5 \times 10^4$ Zellen pro Napf) - in Produktionsmedium suspendiert - zu jedem Näpfchen zugeführt. Jedes Näpfchen erhielt dann 50 µl einer Visna-Viruslösung mit einer Titer von ca. $2{,}5 \times 10^4$ $TCID_{50}$ (TCID = tissue culture infectious dose). Diese Virusdosis entspricht einer MOI (Multiplizität der Infektion) von ca. 0,05.

Unter diesen Infektionsbedingungen resultierte zwischen Tag 5 und Tag 10 in einer Infektionskontrolle ohne Substanz ein virusinduzierter, zytopathischer Effekt. Die infizierten und behandelten Zellen und die Kontrollzellen wurden 7 Tage bei 37° C, 5 % $CO_2$ inkubiert.

Bei Auftreten des virusinduzierten zytopathogenen Effektes in der unbehandelten Viruskontrolle wurden die Kulturen mit Formalin fixiert und anschließend mit einer Giemsa-Lösung gefärbt. Die inhibitorische Konzentration ($IC_{50}$ wurde mikroskopisch als die Konzentration ermittelt, bei der der zytopathische Effekt um 50 % gehemmt wurde: im Vergleich zur unbehandelten Viruskontrolle, die 100 % Zellzerstörung aufwies.

Es wurde gefunden, daß beispielsweise 9-(2,3-Anhydro-β-D-lyxofuranosyl)adenin in einem Konzentrationsbereich von 300 µg/ml bis zu 1 µg/ml die mit Visna-Virus infizierten Zellen vor der virusinduzierten Zellzerstörung schützt.

Die Therapie von HIV-induzierten Erkrankungen erfordert selektiv wirkende Verbindungen mit einer hohen Verträglichkeit. Dies gilt ganz besonders bei HIV-induzierten Erkrankungen, bei denen die Viren die Zellen befallen und zerstören, die für die zelluläre Immunität verantwortlich sind.

Wirksame Verbindungen gegen diese Erreger sollten selektiv die Vermehrung des Virus in diesen Zellen verhindern und eine Neuinfektion frischer Zellen verhindern, ohne daß ihre Funktion zur Abwehr des Virus selbst oder anderer Krankheitskeime wie z.B. opportunistische Infektionen beeinflußt wird.

In weiterführenden Untersuchungen konnte gezeigt werden, daß beispielsweise die Verbindung 9-(2,3-Anhydro-β-D-lyxofuranosyl)adenin diese Bedingungen erfüllt und die Funktion von T-Zellen nicht beeinflußt.

Die erfindungsgemäß zu verwendenden Verbindungen stellen somit wertvolle Wirkstoffe zur Behandlung oder Prophylaxe von Erkrankungen, hervorgerufen durch Retroviren, in der Human- und Tiermedizin dar.

Als Indikationsgebiete in der Humanmedizin können beispielsweise genannt werden:

1. Die Behandlung oder Prophylaxe von menschlichen Retrovirusinfektionen.

2. Für die Behandlung oder Prophylaxe von HIV (Virus der humanen Immundefiziens; früher HTLV III/LAV genannt) verursachten Erkrankungen (AIDS) und den damit assoziierten Stadien wie ARC (AIDS related complex) and LAS (Lymphadenopathie-Syndrom) sowie der durch dieses Virus verursachten Immunschwäche und Encephalopathie.

3. Für die Behandlung oder die Prophylaxe einer HTLV I- oder HTLV II-Infektion.

4. Für die Behandlung oder die Prophylaxe des AIDS-carrier Zustandes (AIDS-Überträger-Zustand).

Als Indikationen in der Tiermedizin können beispielsweise angeführt werden: Infektionen mit

a) Maedi-Visna (bei Schafen und Ziegen)
b) progressives Pneumonievirus (PPV) (bei Schafen und Ziegen)
c) caprine arthritis encephalitis Virus (bei Schafen und Ziegen)
d) Zwoegerziekte Virus (bei Schafen)
e) infektiöses Virus der Anämie (des Pferdes)
f) Infektionen verursacht durch das Katzenleukämievirus.

Zur vorliegenden Erfindung gehören pharmazeutische Zubereitungen, die neben nicht-toxischen, inerten pharmazeutisch geeigneten Trägerstoffen eine oder mehrere Verbindungen der Formel (I) enthalten oder die aus einem oder mehreren Wirkstoffen der Formel (I) bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt. Tabletten, Dragees, Kapseln, Pillen und Granulate können den oder die Wirkstoffe neben den üblichen Trägerstoffen enthalten, wie

(a) Füll- und Streckmittel, z.B. Stärken, Milchzucker, Rohrzucker, Glukose, Mannit und Kieselsäure,
(b) Bindemittel, z.B. Carboxymethylcellulose, Alginate, Gelatine, Polyvinylpyrrolidon,
(c) Feuchthaltemittel, z.B. Glycerin,
(d) Sprengmittel, z.B. Agar-Agar, Calciumcarbonat und Natriumcarbonat,
(e) Lösungsverzögerer, z.B. Paraffin und
(f) Resorptionsbeschleuniger, z.B. quarternäre Ammoniumverbindungen,
(g) Netzmittel, z.B. Cetylalkohol, Glycerinmonostearat,
(h) Adsorptionsmittel, z.B. Kaolin und Bentonit und
(i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lö sungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser stoffe enthalten.

Zur parenteralen Applikation können die Lösungen und Emulsionen auch in steriler und blutisotonischer Form vorliegen.

Suspensionen könne neben dem oder den Wirkstoffen die üblichen Trägerstoffe wie flüssige Verdünnungsmittel, z.B. Wasser, Ethylakohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z.B. Pfefferminzöl und Eukalyptusöl und Süßmittel, z.B. Saccharin, enthalten.

Die Wirkstoffe der Formel (I) sollen in den oben aufgeführten pharmazeutischen Zubereitungen, vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den Verbindungen der Formel (I) auch weitere pharmazeutische Wirkstoffe enthalten.

Die Herstellung der oben aufgeführten pharmazeutischen Zubereitungen erfolgt in üblicher Weise nach

bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignet Zubereitungen kommen Injektionslösungen, Lösungen und Suspensionen für die orale Therapie, Gele, Aufgußformulierunge, Emulsionen, Salben oder Tropfen in Frage. Zur lokalen Therapie können ophthalmo logische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten, Premixe, Konzentrate, Granulate, Pellets, Tabletten, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe, (Kunststoffketten zur lokalen Therapie), Kollagen oder Knochenzement eingearbeitet werden.

Im allgemeinen hat es sich sowohl in der Human- als auch in der Veterinärmedizin als vorteilhaft erwiesen, den oder die Wirkstoffe der Formel (I) in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die erfindungsgemäß zu verwendenden Verbindungen können in den üblichen Konzentrationen und Zubereitungen zusammen mit dem Futter bzw. mit Futterzubereitungen oder mit dem Trinkwasser gegeben werden.

## Ansprüche

1. Arzneimittel enthaltend mindestens ein 1-(2,3-Anhydro-β-D-lyxofuranosyl)purin- oder 9-pyrimidinnucleosid der Formel I

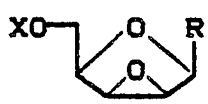

(I)

in welcher

R für eine Gruppe der Formel

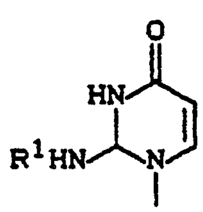

,

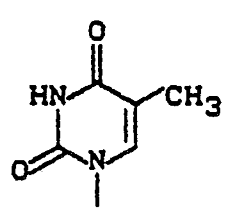

,

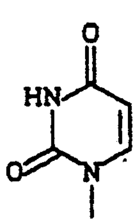

,

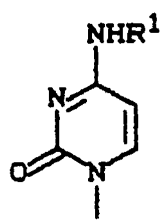

oder

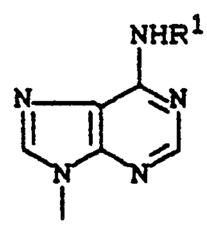

steht,

wobei
$R^1$ Wasserstoff oder Acetyl bedeutet,
und
X für Wasserstoff oder
für geradkettiges $C_1$-$C_7$-Alkylcarbonyl, Isopropylcarbonyl, tert. Butylmethylcarbonyl oder Benzoyl steht, oder ein pharmazeutisch verträgliches Derivat hiervon.

2. Arzneimittel enthaltend mindestens ein 1-(2,3-Anhydro-β-D-lyxofuranosyl)purin- oder 9-pyrimidinnucleosid der Formel (I) in Anspruch 1
in welcher
R für eine Gruppe der Formel

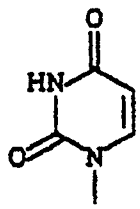 , 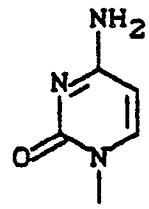 , 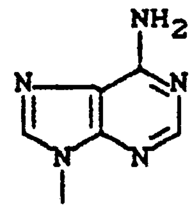 ,

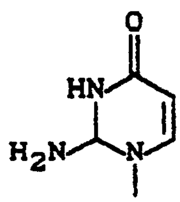 oder 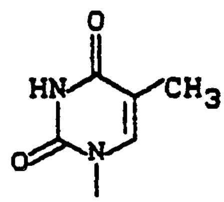 steht,

X für Wasserstoff oder Acetyl, Propionyl, Propylcarbonyl, Isopropylcarbonyl oder Butylcarbonyl steht.

3. Arzneimittel enthaltend mindestens ein 1-(2,3-Anhydro-β-D-lyxofuranosyl)purin- oder 9-pyrimidinnucleosid der Formel (I) nach Anspruch 1
in welcher
R für eine Gruppe der Formel

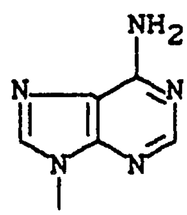 steht,

und
X für Wasserstoff steht.

4. Arzneimittel enthaltend 1-(2,3-Anhydro-β-D-lyxofuranosyl)purin- oder 9-pyrimidinnucleoside.

5. Epoxide der allgemeinen Formel (I)

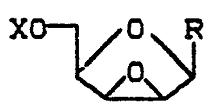 (I)

in welcher
R für eine Gruppe der Formel

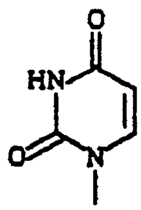 , 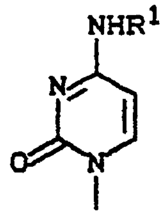 , 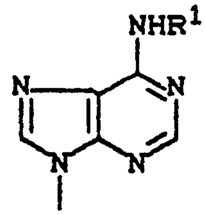 ,

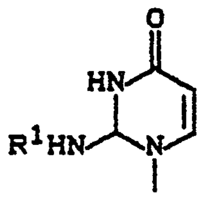 oder 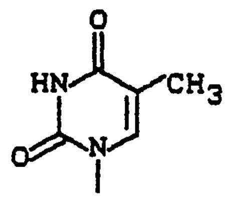 steht,

wobei
$R^1$ Wasserstoff oder Acetyl bedeutet,
und
X für Wasserstoff oder
für geradkettiges $C_1$-$C_7$-Alkylcarbonyl, Isopropylcarbonyl, tert. Butylmethylcarbonyl oder Benzoyl steht,
oder ein pharmazeutisch verträgliches Derivat hiervon,
zur Anwendung in einem Verfahren zur therapeutischen Behandlung des menschlichen oder tierischen Körpers.

6. 1-(2,3-Anhydro-β-D-lyxofuranosyl)purin- oder 9-pyrimidinnucleoside zur Anwendung in einem Verfahren zur therapeutischen Behandlung des menschlichen oder tierischen Körpers.

7. 1-(2,3-Anhydro-β-D-lyxofuranosyl)purin- oder 9-pyrimidinnucleoside der formel (I)

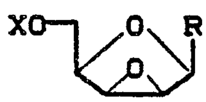 (I)

in welcher
R für eine Gruppe der Formel

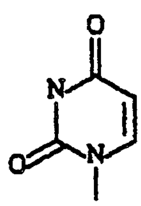 , 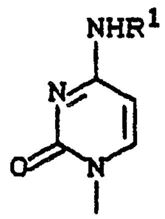 , 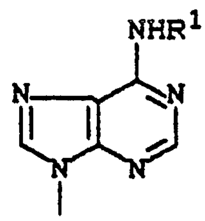 ,

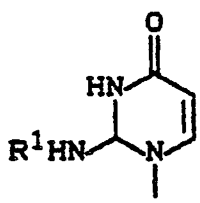 oder 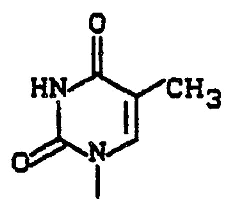 steht,

wobei
$R^1$ Wasserstoff oder Acetyl bedeutet
und
X für Wasserstoff,
für geradkettiges $C_1$-$C_7$-Alkylcarbonyl, Isopropylcarbonyl, tert, Butylmethylcarbonyl oder Benzoyl steht,
oder ein pharmazeutisch verträgliches Derivat hiervon.

8. 1-(2,3-Anhydro-$\beta$-D-lyxofuranosyl)purin- oder 9-pyrimidinnucleoside zur Behandlung von viralen Infektionen.

9. Epoxide der Formel (I)

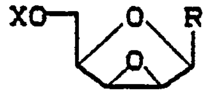

(I)

in welcher
R für eine Gruppe der Formel

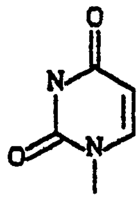 , 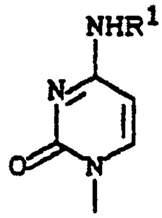 , 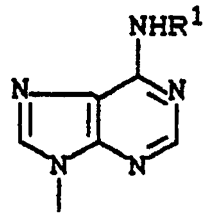 ,

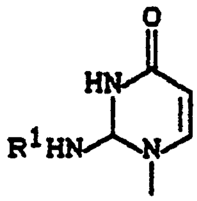 oder 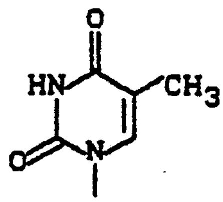 steht,

$R^1$ Wasserstoff oder Acetyl bedeutet,
und
X für Wasserstoff oder
für geradkettiges $C_1$-$C_7$-Alkylcarbonyl, Isopropylcarbonyl, tert. Butylmethylcarbonyl oder Benzoyl steht,
oder ein pharmazeutisch verträgliches Derivat hiervon,
zur Behandlung von retroviralen Infektionen.

10. 1-(2,3-Anhydro-$\beta$-D-lyxofuranosyl)purin- oder 9-pyrimidinnucleoside zur Behandlung von retroviralen Infektionen.

11. Epoxide der Formel (I)

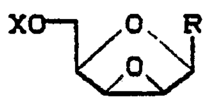

(I)

in welcher
R für eine Gruppe der Formel

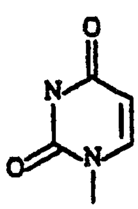 , 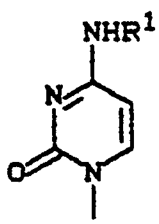 , 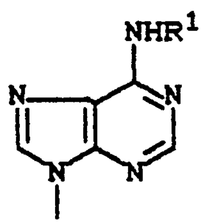 ,

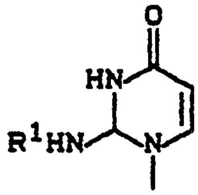 oder 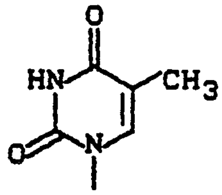 steht,

wobei
$R^1$ Wasserstoff oder Acetyl bedeutet,
und
X für Wasserstoff oder
für geradkettiges $C_1$-$C_7$-Alkylcarbonyl, Isopropylcarbonyl, tert. Butylmethylcarbonyl oder Benzoyl steht,
oder ein pharmazeutisch verträgliches Derivat hiervon,
zur Prophylaxe von viralen Infektionen.

12. 1-(2,3-Anhydro-β-D-lyxofuranosyl)purin- oder 9-pyrimidinnucleoside zur Prophylaxe von viralen Infektionen.

13. Epoxide der Formel (I)

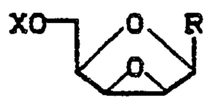 (I)

in welcher
R für eine Gruppe der formel

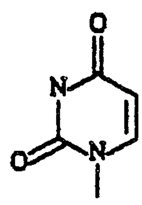 , 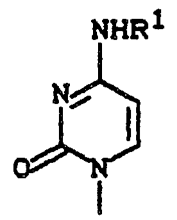 , 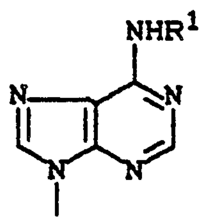 ,

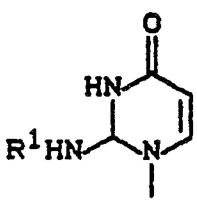 oder 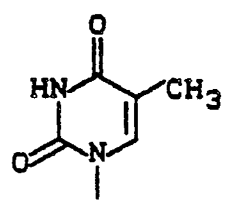 steht,

wobei
$R^1$ Wasserstoff oder Acetyl bedeutet,
und
X für Wasserstoff oder
für geradkettiges $C_1$-$C_7$-Alkylcarbonyl, Isopropylcarbonyl, tert. Butylmethylcarbonyl oder Benzoyl steht,
oder ein pharmazeutisch verträgliches Derivat hiervon,
zur Prophylaxe von retroviralen Infektionen.

14. 1-(2,3-Anhydro-β-D-lyxofuranosyl)purin- oder 9-pyrimidinnucleoside zur Prophylaxe von retroviralen Infektionen.

15. Verwendung von Epoxiden der Formel (I)

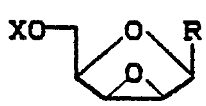 (I)

in welcher
R für eine Gruppe der formel

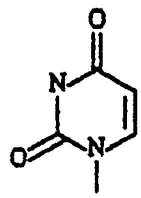 , 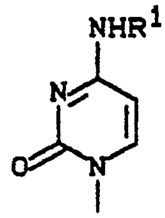 , 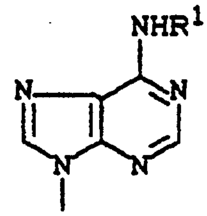 ,

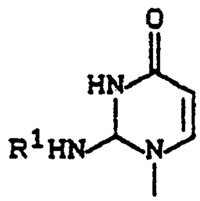 oder 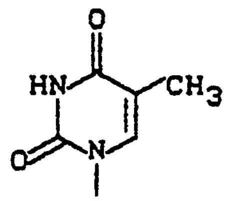 steht,

wobei
$R^1$ Wasserstoff oder Acetyl bedeutet,
und
X für Wasserstoff oder
für geradkettiges $C_1$-$C_7$-Alkylcarbonyl, Isopropylcarbonyl, tert. Butylmethylcarbonyl oder Benzoyl steht,
oder ein pharmazeutisch verträgliches Derivat hiervon,
zur Herstellung von antiviralen Mitteln.

16. Verwendung von 1-(2,3-Anhydro-β-D-lyxofuranosyl)purin- oder 9-pyrimidinnucleosiden zur Herstellung von antiviralen Mitteln.